(19) Europäisches Patentamt
European Patent Office
Office européen des brevets

(11) **EP 2 783 713 B1**

(12) **EUROPÄISCHE PATENTSCHRIFT**

(45) Veröffentlichungstag und Bekanntmachung des Hinweises auf die Patenterteilung:
**05.08.2020 Patentblatt 2020/32**

(51) Int Cl.:
**A61M 1/36** *(2006.01)* **A61M 1/16** *(2006.01)*

(21) Anmeldenummer: **14157989.6**

(22) Anmeldetag: **06.03.2014**

(54) **Rezirkulationsdetektion durch Bolusgabe**

Recirculation detection by bolus administration

Détection de recirculation par bolus

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2013 DE 102013103222**

(43) Veröffentlichungstag der Anmeldung:
**01.10.2014 Patentblatt 2014/40**

(73) Patentinhaber: **B. Braun Avitum AG**
**34212 Melsungen (DE)**

(72) Erfinder: **Ahrens, Jörn**
**34225 Baunatal (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Straße 22 85354 Freising (DE)**

(56) Entgegenhaltungen:
**EP-A1- 0 900 094 EP-A1- 2 292 283 DE-A1-102011 008 482 US-A1- 2006 096 348 US-B2- 7 815 852**

## Beschreibung

Die vorliegende Erfindung betrifft ein (Steuerungs-) Verfahren sowie eine Vorrichtung zur (maschineninternen) Erfassung eines Rezirkulationszustands durch/unter Bolusgabe.

Hintergrund der Erfindung

Die Dialyse ist ein Blutreinigungsverfahren, das bei Nierenversagen als Ersatzverfahren zum Einsatz kommt. Die Dialyse ist neben der Nierentransplantation die einzige und damit wichtigste Nierenersatztherapie bei chronischem Nierenversagen und eine der Behandlungsmöglichkeiten bei akutem Nierenversagen. Unter Dialyse wird dabei ein Stoffaustausch über eine Membran verstanden, wobei auf der einen Membranseite Blut/Plasma und auf der anderen Seite der Membran eine Dialysierflüssigkeit anliegt bzw. im Gegenstromprinzip entlang strömt.

Während einer Behandlung wird hierfür über einen Patientenzugang Blut aus dem Patienten gepumpt, im Dialysator (Filter) an der Dialysemembran vorbeigeführt und in gereinigtem Zustand dem Patienten wieder zurückgegeben. Giftstoffe bzw. urämische Toxine (Stoffwechselabbauprodukte) und niedermolekulare Stoffe (membrangängige Stoffe) werden in einem Hämodialyse- oder Hämodiaflitrations-Betriebsmodus der Dialysemaschine aus dem Blut durch Konzentrationsgradienten (Diffusion) und/oder Ultrafiltration (Konvektion) über die Membran auf die andere Filterseite in die Dialysierflüssigkeit gefördert und dadurch entfernt. Der Dialysator wird dabei ständig von frischer Dialysierflüssigkeit durchströmt (ca. 500 ml/min).

Die Hämodialysebehandlung wird in der Regel ca. 4 - 5 Stunden (Nachtdialyse bis 8 Stunden) pro Behandlung und mindestens dreimal in der Woche durchgeführt (abhängig von Körpergewicht, Nierenrestfunktion, Herzleistung). Patienten, die Heimhämodialyse durchführen, vermeiden das problematische längere Behandlungsintervall am Wochenende und dialysieren häufiger, im Regelfall alle zwei Tage oder sogar täglich.

Ein entscheidender Einflussfaktor auf die Qualität der Dialyse ist der Blutfluss. Je höher dieser nämlich ist, desto besser ist im Allgemeinen auch das Behandlungsergebnis. Allerdings kann es vorkommen, dass im patientenimplantierten Dialyseshunt zur Blutentnahme und Rückführung Probleme auftreten, insbesondere wenn der Blutfluss im extrakorporalen System hoch ist. Die genaue Höhe des Blutflusses, bei dem diese Probleme auftreten, hängt von der Qualität des Patientenzugangs und dem Blutfluss durch den Shunt sowie dessen Unversehrtheit (keine Stenosen oder Aneurismen) ab.

In der Dialyse kann es daher durch eine Veränderung des Zugangs, Stenosen oder bei einem zu geringen Shuntfluss zu einer sogenannten Rezirkulation im Shunt kommen. Eine Rezirkulation verursacht den direkten Rückfluss von gereinigtem Blut aus dem venösen Zugang direkt wieder in den arteriellen Zugang zum Dialysator, wodurch die Dialyseeffektivität deutlich reduziert wird. Daher sollte / muss sichergestellt werden, dass sich die Dialyseleistung während der gesamten Therapie nicht verringert und somit eine effektive Dialyse gewährleistet werden kann.

Um eine Rezirkulation im Shunt während der Dialysebehandlung zu erkennen, gibt es im Stand der Technik bereits verschiedene Steuerungsverfahren, die unabhängig zum medizinischen Dialyse - Behandlungsverfahren am Patienten ablaufen, wie sie nachfolgend prinzipiell dargestellt werden.

Stand der Technik

Die Bestimmung von Rezirkulation im Shunt des Patienten wurde demzufolge bereits im Stand der Technik durch verschiedene Verfahren vorgestellt. So wird bei an sich bekannten Dialysegeräten die Erkennung der Rezirkulation beispielsweise durch eine sensorisch durchgeführte Temperaturkontrolle (EP 0 900 094) realisiert. Dazu wird das extrakorporal geführte Blut im venösen Leitungsabschnitt mit einem Bolus in Form eines Temperaturpulses versehen. Die Temperaturmessung im arteriellen Leitungsabschnitt / -system ermöglicht dabei auf Basis einer Temperaturänderung den Rückschluss auf den prozentualen Anteil der Rezirkulation.

Des Weiteren wird im einschlägigen Stand der Technik eine Messung der Inline-Dialyse angeboten, welche auf Basis einer Leitfähigkeitsmessung erfolgt und ebenfalls mit der Gabe eines geeignetes Bolus arbeitet. Hierbei wird die Rezirkulation im Shunt eines Dialysepatienten durch die Messung der Leitfähigkeit ermittelt. Auch bei diesem Verfahren wird mittels der Dialysierflüssigkeit in den venösen Schlauchabschnitt ein Elektrolytbolus verabreicht, der die Leitfähigkeit der Flüssigkeit modifiziert. Durch die Bestimmung der Leitfähigkeit in dem dem Dialysator nachgeschalteten Dialysierflüssigkeitsabfluss kann somit auf den Grad der Rezirkulation (maschinenintern) zurückgeschlossen werden (US 7,815,852).

Die WO 98/32477 beschreibt ein Verfahren zur Messung der Rezirkulation R, d. h. der Summe der Fistelrezirkulation ($R_A$) und der kardiopulmonalen Rezirkulation $R_{CP}$. Bei dem bekannten Verfahren wird eine physikalische oder chemische Kenngröße der Dialysierflüssigkeit im Dialysierflüssigkeitsweg stromauf des Dialysators verändert, die zu einer Änderung der physikalischen oder chemischen Kenngröße auf der Blutseite führt. Die Änderung der Kenngröße auf der Blutseite führt zu einer Änderung der Kenngröße der Dialysierflüssigkeit stromab der Dialysierflüssigkeitskammer des Dialysators. Zur Bestimmung der Rezirkulation wird die Kenngröße im Dialysierflüssigkeitsweg stromab des Dialysators gemessen und aus dem zeitlichen Verlauf der Änderung der Kenngröße wird die Rezirkulation R bestimmt. Als physikalische oder chemische Kenngröße kann die Dialysierflüssigkeits-IonenKonzentration, beispielsweise die Na-Konzentration der Dialysierflüssigkeit, oder auch die Temperatur der Dialysierflüssigkeit verändert und gemessen werden.

Darüber hinaus kann Rezirkulation prinzipiell auch mit Hilfe eines Hämatokrit - Sensors bestimmt werden. Dabei wird der Hämatokrit des Blutes durch die Gabe eines definierten Bolus im venösen Leitungsabschnitt geändert. Durch die Bestimmung des Hämatokrits im arteriellen Leitungssystem ist die Bestimmung der Rezirkulation möglich. Technische Vorschläge, die auf diesem Prinzip basieren, sind beispielsweise in der US 5,685,989 offenbart. Diese bekannten Produkte beruhen entweder auf der Messung der Rezirkulation mittels elektromagnetischer Strahlung im sichtbaren Bereich, oder auf der Messung der Rezirkulation durch Ultraschall.

Die beschriebenen Verfahren/Produkte können die Rezirkulation im Patientenzugang auf unterschiedlichen Wegen bestimmen. Dabei sei ausdrücklich darauf hingewiesen, dass sich die betreffenden Verfahren auf interne Steuerungsprozeduren zur Optimierung der Leistungsfähigkeit des Dialysesystems / der Dialysemaschine beziehen und nicht auf eine besondere medizinische Anwendung der Dialysemaschine zur Ausführung einer Patientenbehandlung. D.h. die genannten Maschinensteuerungsprozesse laufen automatisch unabhängig von einer Patientenbehandlung zur Aufrechterhaltung der internen Maschinenfunktion.

Des Weiteren existiert im einschlägigen Stand der Technik beispielsweise gemäß der DE 699 16 053 T2 ein weiteres Messverfahren zur Bestimmung von Abfallprodukten in maschineninternen Dialyseflüssigkeiten während der Dialysebehandlung unter Anwendung einer spektralphotometrischen Messung insbesondere mittels UV - Licht. Konkreter ausgedrückt, beruht dieses Messverfahren gemäß der DE 699 16 053 T2 auf dem allgemeinen Prinzip der Fotometrie, wonach eine Lichtquelle eingesetzt wird und als Detektoren sogenannte Fotosensoren dienen. Die Lichtquelle emittiert dabei ein Lichtsignal mit einer Wellenlänge von 180 - 380nm. Diese Wellenlänge wird von harnpflichtigen Substanzen (urämischen Toxinen) absorbiert.

Alle diese bekannten Verfahren können die maschinenspezifische Dialyseeffektivität durch die Erkennung einer Rezirkulation im Shunt punktuell feststellen, aber folgende Beschränkungen sind gegeben:
Die Bolusgabe erfolgt in der Regel (mit Ausnahme der spektralphotometrischen Messung) auf der Blutseite im venösen Abschnitt, wobei die Messung anschließend ebenfalls blutseitig im arteriellen Abschnitt durchgeführt werden muss. Störungen zwischen den Messintervallen bleiben zunächst verborgen, bzw. können nicht identifiziert werden. Die Behandlung wird durch die punktuellen blutseitigen Messungen negativ beeinflusst. Da die Messungen blutseitig und nicht dialysierflüssigkeitsseitig erfolgen, müssen diese durch geeignetes Fachpersonal ausgelöst und begleitet werden.

Kurzbeschreibung der Erfindung

Angesichts dieser Problematik ist es die Aufgabe der vorliegenden Erfindung, eine Online - Überwachung der Dialysetherapie / -sitzung bzw. der Dialyseeffektivität zu ermöglichen und die Messung im Dialysierflüssigkeitskreislauf (Dialysierflüssigkeitsseite), d.h. ausschließlich maschinenintern und damit losgelöst vom Patienten und damit von der Patientenbehandlung als solche auszuführen. Ein Ziel der Erfindung soll es ferner sein, dass die Messung auf jener Sensorik basiert, welche ohnehin bereits in der Dialysemaschine allgemein vorhanden ist.

Diese Aufgabe wird durch ein maschineninternes (steuerungstechnisches) Mess- / Bestimmungsverfahren mit den Verfahrensschritten gemäß dem Patentanspruch 1 sowie durch eine Vorrichtung mit den Merkmalen gemäß dem Patentanspruch 6 gelöst. Vorteilhafte Ausgestaltungen und/oder Weiterbildungen der vorliegenden Erfindung sind Gegenstand der Unteransprüche.

Der Kern der vorliegenden Erfindung besteht gemäß einem Aspekt in der Bereitstellung eines (maschineninternen) (Steuerungs-) Verfahrens zur dialysierflüssigkeitsseitigen Erfassung / Bestimmung einer Rezirkulation durch / unter blutseitige(r) Bolusgabe mit mindestens den folgenden Verfahrensschritten bzw. Steuerungsprozessschritten:

Spektralphotometrisches Messen eines ersten (UV-) Licht - Absorbanzwerts (oder Absorbtionswerts) einer von einem Dialysator abfließenden Dialysierflüssigkeit vor Bolusgabe zu einem Zeitpunkt während einer Dialysebehandlung,
Venöse Bolusgabe (z.B. NaCl oder jede andere Substanz, die keine oder eine starke und bekannte Absorption bei 280nm Licht - Wellenlänge hat) mit vorbekanntem (adaptive Bestimmung auf Basis des Blutflusses oder fix) Volumen am / unmittelbar vor dem venösen Zugang,
Spektralphotometrisches Messen eines zweiten (UV-) Licht - Absorbanzwerts (oder Absorbtionswerts) der abfließenden Dialysierflüssigkeit nach Bolusgabe vorzugsweise Bestimmen der zwischen der Messung des ersten Absorbanzwerts (ad) und des zweiten Absorbanzwerts (bd) vergangenen Zeitspanne und
Ermitteln einer Absorbanzwertänderung (oder Absorbtionswertänderung) zwischen dem ersten und dem zweiten gemessenen Absorbanzwerten (oder Absorbtionswerten) hervorgerufen durch eine (verdünnende) Bolus - Präsenz in der Dialysierflüssigkeit infolge von Rezirkulation ggf. als Grundlage für eine nachfolgende Rezirkulationsquantifizierung (d.h. die Präsenz einer erfassten / ermittelten Absorbanzdifferenz (oder Absorbtionsdifferenz) in der Dialysierflüssigkeit infolge der Rezirkulation kann dann als Grundlage einer nachfolgenden Rezirkulationsquantifizierung dienen).

Durch dieses Verfahren lässt sich auf einfache Weise unter Verwendung eines UV-Sensors am dialysierflüssigkeitsseitigen Dialysatorausgang eine Absorbanzwertänderung (oder Absorbtionswertänderung) über einen bestimmten Zeitraum während der Dialysebehandlung erkennen und ggf. auch quantifizieren, der aufgrund des venös gegebenen Bolus (vorbekannter Menge) verursacht worden ist. Da dieser venös gegebene Bolus nur durch einen Rezirkulationseffekt in den Dialysator (teilweise) gelangt sein kann und dort zu einer Konzentrationsverringerung an messbaren urämischen Toxinen führt, ist diese Konzentrationsverringerung indirekt ein Maß für den Rezirkulationsgrad zum Messzeitpunkt.

Gemäß einer Weiterbildung der vorliegenden Erfindung sind die folgenden Verfahrensschritte noch vorgesehen:

Integralbildung über den Absorbanz (oder Absorbtions) - Änderungsverlauf für die ersten und zweiten Absorbanzwerte (oder Absorbtionswerte) vor und nach dem Bolus, wodurch zwei Integralflächen errechnet werden,
Differenzbildung zwischen den beiden Integralwerten / Integralflächen vor und nach dem Bolus,
Erkennen eines (zunächst nicht quantifizierten) Rezirkulationszustands im Fall einer Differenz (C) ungleich null.

In anderen Worten ausgedrückt betrifft das erfindungsgemäße Steuerungsverfahren der Dialysemaschine prinzipiell

1) ein Einholen von Detektorsignalen, ausgegeben von einem in der Regel vorhandenen UV-Sensor im Dialysierflüssigkeitsabschnitt der Dialysemaschine vor und nach einer Bolusgabe, z.B. NaCl oder jede andere Substanz, die keine oder eine starke und bekannte Absorption bei 280nm Licht - Wellenlänge hat,
2) ein Erfassen einer Signaländerung (bspw. durch eine Verringerung der Harnsäurekonzentration im arteriellen Zugang durch den Bolus) und vorzugsweise
3) ein Bestimmen des Vorhandenseins einer Rezirkulation auf der Grundlage einer aus der Signaländerung ermittelten Signalwertdifferenz.

Vorzugsweise ist der vorstehend genannte Steuerungsverfahrensschritt 3) untergliedert in

4) eine Integralbildung über den Signalverlauf vor und nach dem Bolus,
5) Ermitteln einer Differenz zwischen den beiden Integralwerten für den Signalverlauf vor und nach dem Bolus und
6) Erkennen auf das Vorhandensein einer Rezirkulation für den Fall einer Integralwertdifferenz ungleich 0.

Durch diese Verfahrensschritte ergibt sich die Möglichkeit, das Vorhandensein von Rezirkulation zu bestimmen, um dann ggf. weitere Verfahrensschritte für dessen Quantifizierung (Rezirkulationsgrad) einzuleiten. Diese weiteren Verfahrensschritte können die Folgenden umfassen:

- Umrechnen des einen Integralwerts / der einen Integralfläche vor der Bolusgabe sowie der Integralwert-/Integralflächendifferenz in zugehörige Absorbanzwerte
- Berechnen der arteriellen Bolusmenge (entspricht jenem Bolusteil, der infolge Rezirkulation in die Dialysierflüssigkeit übergegangen ist) aus dem Verhältnis der umgerechneten Absorbanzwerte und
- Berechnen des Rezirkulationsgrads aus dem Verhältnis der errechneten arteriellen Bolusmenge zu der vorbekannten venösen (zugegebenen) Bolusmenge.

Mit diesen letzten Verfahrensschritten ist dann eine Quantifizierung einer ggf. vorliegenden Rezirkulation lediglich unter Zuhilfenahme zumindest eines UV - Sensors gegeben.

Gemäß einem anderen Aspekt der vorliegenden Erfindung wird eine Dialysemaschine mit einer Vorrichtung zur dialysierflüssigkeitsseitigen Erfassung einer Rezirkulation durch blutseitige Bolusgabe unter Implementierung des Verfahrens gemäß vorstehender Definition vorgeschlagen mit zumindest einer Lichtquelle, die Licht mit einer Wellenlänge von 250 - 300nm und vorzugsweise 280nm emittiert, mindestens einem UV-Sensor vorzugsweise unmittelbar am dialysierflüssigkeitsseitigen Abfluss eines Dialysators und einer Recheneinheit, welche Sensorsignale bezüglich der UV - Absorbanz (oder UV - Absorption) der Dialysierflüssigkeit vor und nach einer blutseitig venösen Bolusgabe von dem zumindest einen UV - Sensor erhält und welche vorzugsweise eine Intergralbildung über den Signalverlauf für eine bestimmte Zeitspanne vor und nach der Bolusgabe durchführt.

Auch bei der vorliegenden Erfindung wird als Messverfahren das aus der DE 699 16 053 T2 bereits bekannte Messprinzip gemäß vorstehender Beschreibung angewandt. Als Lichtquelle, dient vorliegend eine LED, welche, wie bereits vorstehend ausgeführt, Licht mit einer Wellenlänge von ca. 280nm emittiert, das vorliegend von zwei Fotodetektoren (UV - Sensoren) erfasst wird. Der zumindest eine Sensor befindet sich im Abfluss (unmittelbar) hinter dem Dialysator der Dialysemaschine auf der Dialysierflüssigkeitsseite und misst während der Dialyse die Absorbanz (oder Absorption) im Dialysierflüssigkeitsabfluss kontinuierlich oder getaktet.

Durch das erfindungsgemäße Verfahren wird eine Online - Überwachung der Therapie bzw. der Dialyseeffektivität ermöglicht, wobei die Messung nicht am Patienten sondern maschinenseitig im Dialysierflüssigkeitskreislauf stattfindet. Ferner basiert die Messung (ausschließlich) auf jener Sensorik, die bereits in der Dialysemaschine vorhanden ist.

Figurenbeschreibung

Die Erfindung wird nachstehend anhand eines bevorzugten Ausführungsbeispiels unter Bezugnahme auf die begleitenden Zeichnungen näher erläutert. Es zeigen

Fig. 1 den Prinzipaufbau bzw. die Strömungsverhältnisse in einer Dialysemaschine gemäß der Erfindung ohne Rezirkulation,

Fig. 2 den Prinzipaufbau bzw. die Strömungsverhältnisse in einer Dialysemaschine gemäß der Erfindung mit Rezirkulation,

Fig. 3 den Blutkreislauf im Shuntbereich ohne Rezirkulation bei Bolusgabe,

Fig. 4 den Blutkreislauf im Shuntbereich mit Rezirkulation bei Bolusgabe und

Fig. 5 den qualitativen Verlauf eines UV - Sensorsignals bei venöser Bolusgabe am Shunt und Rezirkulation.

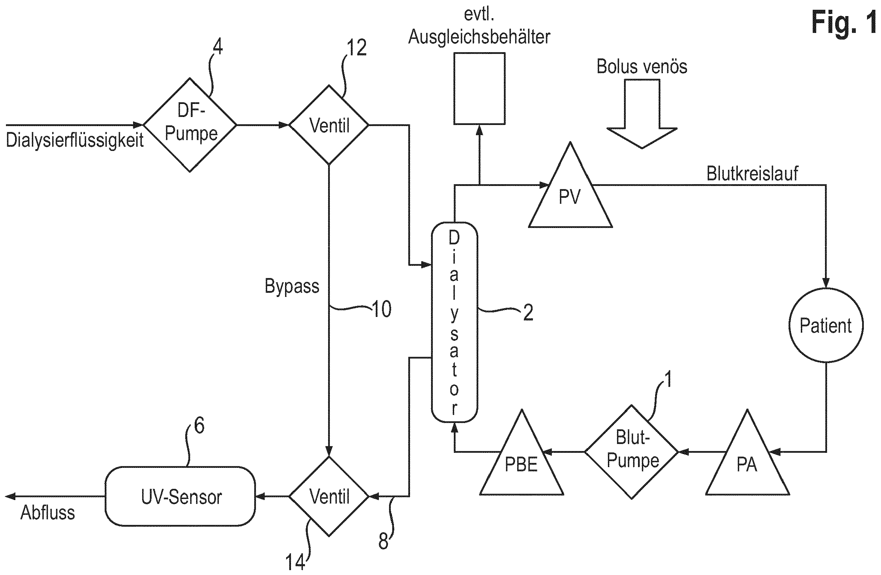

Aus der Fig. 1 ist der prinzipielle Aufbau einer Dialysemaschine (Dialysierflüssigkeitsströmung) ohne eine Rezirkulation gemäß der vorliegenden Erfindung dargestellt. Demnach fördert eine Blutpumpe 1 Blut aus dem Körper eines Patienten über (im Folgenden nicht näher spezifizierte) Druckmesseinrichtungen in einen Dialysator 2, in welchen maschinenseitig sowie in Gegenstromrichtung zum Blut eine Dialysierflüssigkeit mittels einer Dialysierflüssigkeitspumpe 4 gepumpt wird. Blut und Dialysierflüssigkeit sind durch eine semipermeable Membran (nicht näher dargestellt) voneinander getrennt, die für urämische Toxine in Richtung Dialysierflüssigkeit durchlässig ist. Die mit urämischen Toxinen angereicherte Dialysierflüssigkeit wird dann aus dem Dialysator 2 abgeführt.

Zumindest ein UV - Sensor 6 befindet sich in/an einer Abflussleitung 8 stromab zum Dialysator 2 und misst während einer Dialysetherapie die (UV - Licht) Absorbanz im Abfluss (Dialysierflüssigkeit) kontinuierlich. Zu Beginn wird zur Kalibrierung des Systems (Sensorik) ein Detektor 0 - Wert mit reiner Dialyseflüssigkeit (ohne urämische Toxine) ermittelt und zwar vorzugsweise zu einem Zeitpunkt, zu welchem der Patient noch nicht an die Dialysemaschine angelegt ist oder in dem der Dialysator 2 über eine Umgehungsleitung 10 kurzgeschlossen wird, welche an zwei Ventilen 12, 14 mit dem Ein- und Ausgang des Dialysators 2 fluidverbindbar ist. In letzterem Fall (Bypass - Lösung) ist der UV - Sensor 6 stromab zu der Ungehungsleitung 19 am Dialysatorabfluss 8 platziert. Anschließend wird die Dialysierflüssigkeit wieder über den Dialysator 2 geleitet und kontinuierlich oder getaktet vom UV - Sensor gemessen. Die dabei erzeugten Sensorsignale Detektor i werden dann einer nicht weiter dargestellten Recheneinheit zugesandt, welche hieraus den aktuelle (UV - Licht) Absorbanzwert wie Folgt ermittelt:

$$A = \log_{10}\left(\frac{Detektor_0}{Detektor_i}\right)$$

Wie bereits vorstehend ausgeführt wurde, ist der Detektor 0 - Wert das zu Beginn der Dialysebehandlung kalibrierte Level, das mit einer Dialysierflüssigkeit ohne die zu messenden Substanzen durchgeführt wird. Der Dialysierflüssigkeitsfluss, der Blutfluss und der verwendete Dialysator 2 beeinflussen die Absorbanz und somit auch die vorherrschende Clearance (Anteil des Blutflusses zum Gesamtblutfluss, der vollständig von urämischen Toxinen gereinigt ist), die maßgeblich für die (UV - Licht) Absorbanz im Abfluss ist. Dabei werden die entzogenen Giftstoffe von der Blutseite durch den Dialysierflüssigkeitsfluss verdünnt und dann im Abfluss durch den zumindest einen UV - Sensor 6 gemessen.

Wie bereits eingangs beschrieben wurde, wird während einer Rezirkulation im Shunt schon gereinigtes Blut aus dem venösen Zugang wieder in den arteriellen Zugang zurückgesaugt. Die Rezirkulation (in %) ist dabei das Verhältnis zwischen gereinigtem Blut und dem aktuellen Blutfluss. Dadurch wird nicht die tatsächliche im Patienten aktuell vorhandene Konzentration an Giftstoffen zum Dialysator 2 befördert, sondern nur eine Verdünnung mit bereits gereinigtem Blut, wodurch sich die Dialyseeffektivität natürlich verringert.

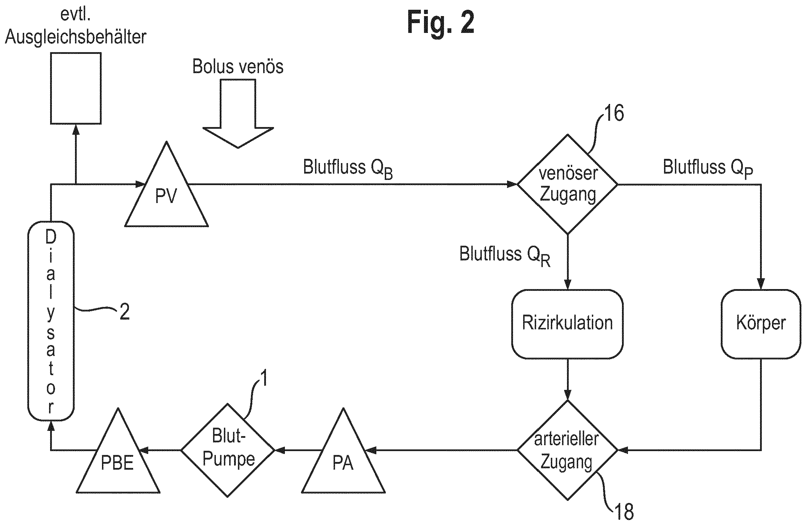

In der Fig. 2 ist die erfindungsgemäße Dialysemaschine in einem Rezirkulationszustand prinzipiell dargestellt. Demnach wird gereinigtes Blut in den venösen Zugang 16 abgeführt und gleich im Anschluss daran parallel zum arteriellen Patientenblut erneut in den arteriellen Zugang 18 eingesaugt. Der in Fig. 2 gekennzeichnete Blutfluss parallel zum Patientenkörper bezeichnet hierbei die Rezirkulation.

Sie lässt sich errechnen aus:

$$\text{Rezirkulation R } [\%] = \frac{Q_R}{Q_B}$$

$$Q_B = Q_R + Q_P$$

mit

    QB = Gesamtblutfluss
    QR = Rezirkulationsblutfluss
    QP = Patientenblutfluss

Ausgehend von den vorstehenden Grundbetrachtungen lässt sich die absolute Rezirkulation auf die nachfolgend beschriebene Weise bestimmen:
Eine getriggerte Messung zur Bestimmung einer absoluten Rezirkulation in einem Shunt erfolgt erfindungsgemäß durch die Zugabe eines Bolus in den venösen Zugang. Dieses Verfahren kann auch bei einer nicht plötzlich auftretenden Rezirkulation (kontinuierlich) durchgeführt werden und bestimmt/berechnet den Absolutwert der Rezirkulation.

Die Bolusgabe kann zunächst auf verschiedenen Wegen mittels Spritze, Rückfiltration, Substitutionspumpe, etc. in den venösen extrakorporalen Kreislauf erfolgen und muss nur (unmittelbar) vor dem venösen Zugang zugegeben werden. Der Bolus selbst sollte dabei vorzugsweise aus einer nicht absorbierenden Substanz (z.B. NaCl) bestehen. Sofern die Konzentration des Bolus bekannt ist und diese Konzentration ebenfalls auf den UV- Sensor kalibriert ist, kann alternativ auch mit einer absorbierenden Bolussubstanz gearbeitet werden.

Grundsätzlich wird eine bestimmte Bolusmenge (Volumen) QBven beispielsweise 10ml an NaCl venös dem Patienten zugegeben. Die Menge des Bolus kann hierfür adaptiv an den Blutfluss angepasst und somit optimal berechnet werden oder durch eine fixierte Menge definiert sein. Vorzugsweise erfolgt die Bolusgabe ferner bei einem gestoppten Blutfluss, wobei aber auch eine Bolusgabe parallel zum fließenden Blutfluss erfolgen kann.

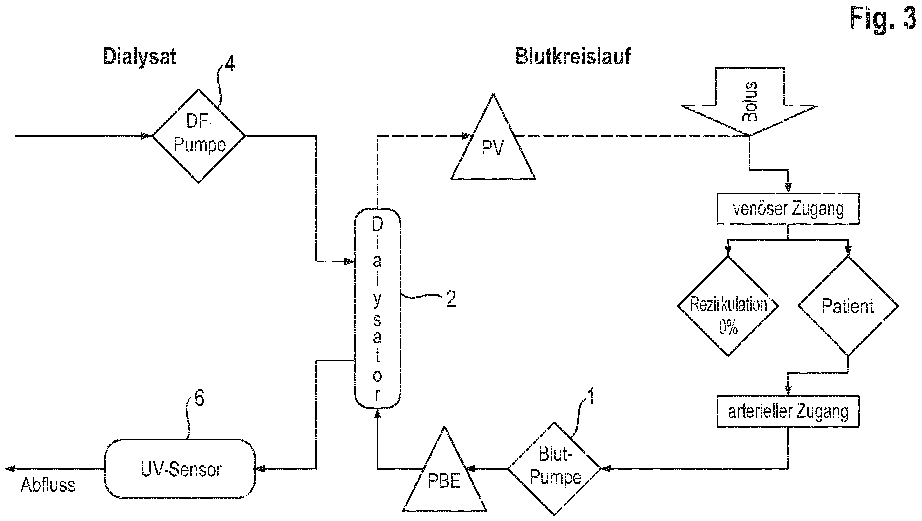

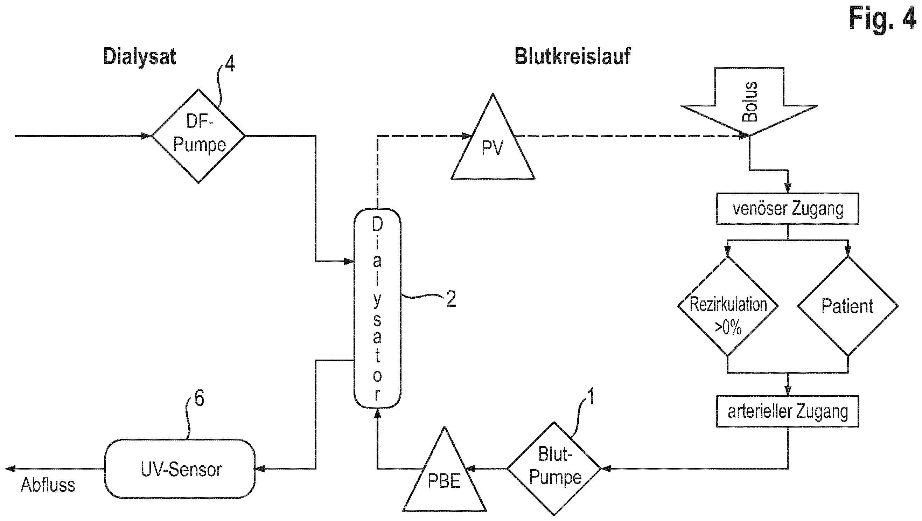

Die Fig. 3 und 4 zeigen das Systemverhalten mit und ohne Rezirkulation bei einem Bolus gemäß der vorstehenden Definition. In Fig. 3 ist jenes Verhalten gezeigt, bei welchem keine Rezirkulation auftritt, wohingegen Fig. 4 das Verhalten mit einer Rezirkulation und der verdünnten Flüssigkeit im arteriellen Zugang darstellt. Demzufolge gelangt der Bolus gemäß der Fig. 3 über den venösen Zugang ausschließlich in den Patientenkörper und von dort in nicht mehr wahrnehmbarer Konzentration in den arteriellen Zugang. In diesem erfasst der UV - Sensor dann keine Absorbanzwertänderung unmittelbar im Anschluss an eine Bolusgabe.

Anders verhält sich das System gemäß der Fig. 4. Dort gelangt der Bolus nicht nur ausschließlich in den Patientenkörper, sondern anteilsmäßig auch unmittelbar in den arteriellen Zugang. Dies führt zu einer raschen Verringerung des dialysierflüssigkeitsseitig gemessenen Absorbanzwerts gegenüber jenem Wert, wie er zeitlich vor der Bolusgabe vorgeherrscht hat, infolge einer Rezirkulation größer null (%).

D.h., sofern keine Rezirkulation im Shunt vorhanden ist, erfolgt vor und nach der Bolusgabe keine Signaländerung bezüglich der Absorbanz bzw. des UV - Sensorsignals, da nur ungereinigtes Blut durch den arteriellen Zugang aufgenommen wird, wohingegen sich der Bolus im Patientenkörper verteilt/verflüchtigt. Eine Rezirkulation im Shunt hingegen (siehe Fig. 4) verursacht eine direkte Signaländerung am UV - Sensor durch eine Verringerung der Konzentration an urämischen Toxinen (Harnsäurekonzentration) im arteriellen Zugang und damit in der Dialysierflüssigkeit. Dabei wird bereits gereinigtes Blut und auch der Bolus aus dem venösen Zugang wieder direkt (ohne über den Patientenkörper zu gehen) durch den arteriellen Zugang aufgenommen und am Dialysator vorbei geführt. Dies führt zu einer geringeren entfernten Menge an urämischen Toxinen und somit auch zu einer geringeren Harnsäurekonzentration (Konzentrationssprung) im Dialysierflüssigkeisabfluss.

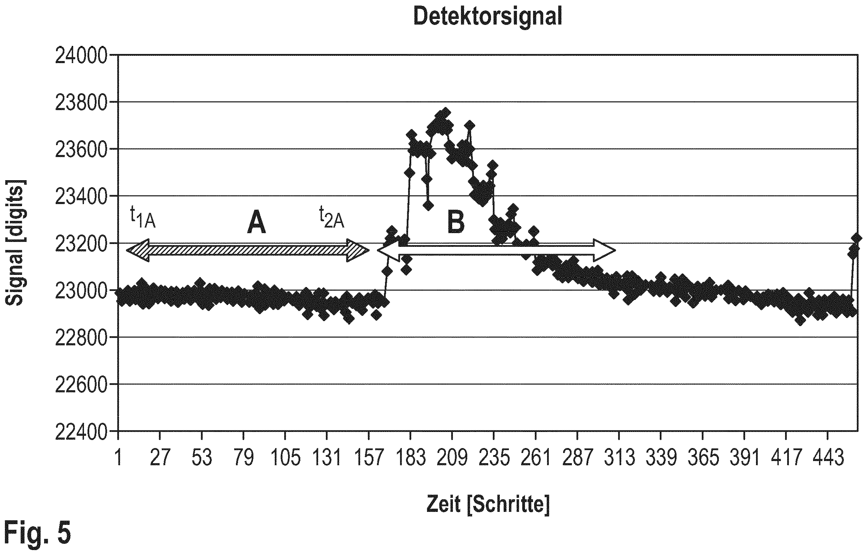

Ein qualitativer Signalverlauf am UV - Sensor im Fall einer Bolusgabe bei Rezirkulation ist in der Fig. 5 dargestellt. Demnach zeigt das Erfassungssignal vor der Bolusgabe einen im Wesentlichen konstanten Verlauf, um dann unmittelbar nach der Bolusgabe einen Signalsprung auszuführen, der eine im Wesentlichen abrupte Konzentrationsänderung an urämischen Toxinen in der Dialysierflüssigkeit anzeigt. Sobald der Bolus den Dialysator passiert hat, kehrt das Sensorsignal allmählich wieder auf seinen Wert vor der Bolusgabe zurück.

Eine Integralbildung über den Signalverlauf für eine bestimmte Zeitspanne vor und nach der Bolusgabe bildet dabei die Grundlage für die quantitative Signalauswertung durch die nicht weiter dargestellte Recheneinheit. Die Messgröße für die Detektion der Rezirkulation mit dem dargestellten UV - Sensor ist demnach (alleinig) das Detektorsignal, das sich aufgrund einer Regelung nur mit der Harnsäurekonzentration und somit mit der proportionalen Absorbanz in der Dialysierflüssigkeit ändert.

Um den genauen Rezirkulationsgrad zu messen / berechnen muss also die Berechnung durch die Absorbanz erfolgen, da diese direkt proportional zur Konzentration ist. Der zugegebene Bolus erreicht gemäß der Fig. 4 demnach vermischt/verdünnt den venösen Zugang und dient als Ausgangssubstanz für die Rezirkulationsbestimmung. Mit Hilfe der zwei genannten Integralsflächen A, B gemäß der Fig. 5 ist es nun möglich, den Rezirkulationsgrad (quantitativ) zu errechnen/bestimmen.

Die Fläche A spiegelt den stationären Wert wieder, der sich in der Dialysierflüssigkeit ohne Boluseinfluss eingependelt hat. Durch den Bolus wird dieses System verändert und für einen kurzen Zeitraum die Konzentration verändert. Diese Änderung kann nunmehr über die Verhältnisse der Absorbanzen bestimmt werden. Voraussetzung dabei ist jedoch die Kenntnis über das Volumen QBven des Bolus, der in den venösen Zugang gegeben wurde.

Die Berechnung erfolgt dann durch die Bestimmung der Integralflächen A, B. Die Messlänge der Integralflächen muss hierfür nicht notwendiger Weise gleich lang sein. Bei unterschiedlichen Längen muss jedoch die Bolusmenge durch die Messlänge berechnet werden. Aus der Differenz der beiden Integralsflächen

$$C = B - A$$

wird die Differenzfläche C gebildet. Diese ist durch die Zugabe des Bolus entstanden und ist direkt proportional zum Bolus (rezirkulierte Menge). D.h., sofern A = B ist, tritt keine messbare Rezirkulation im System auf. Die Kenntnis der Menge QBven über den zugegebenen Bolus ermöglicht nunmehr die Berechnung der Rezirkulation. Dies ist das Verhältnis aus der Menge des eingegebenen venösen Bolus QBven und des davon arteriell aufgenommenen (rezirkulierten) Teils QBart.

$$\text{Rezirkulation} = QBart\ /\ QBven = QR\ /\ QB$$

Die arterielle Bolusmenge QBart ist jene Bolusmenge in ml, die dem Anteil im arteriellen Zugang durch Rezirkulation entspricht. Die Bestimmung erfolgt durch die Berechnung des Verhältnisses der Integralflächen A, B und des effektiven Blutflusses.

Die Flächen A bis C werden aus dem Detektorsignal berechnet. Die Anpassung erfolgt z.B. durch eine Gaus Funktion 7. Ordnung oder eine andere verwendbare mathematische Funktion zur Flächenbestimmung.

$$\text{Fläche}\_C = \int_{t_1}^{t_2} \left( \sum_1^7 \left( a_i \bullet e^{-\left(\frac{x_i - b_i}{c_i}\right)^2} \right) \right)$$

Die Berechnung eines C - Detektorwerts und eines A - Detektorwerts der jeweiligen Flächen C und A erfolgt ferner durch die Mittelwertbildung der jeweiligen Fläche über den bestimmten Messzeitraum. Dadurch wird die effektive Fläche berechnet und man kann über die Flächen die Änderung des Mischungsverhältnisses (Dialysierflüssigkeit / urämische Toxine) in der Dialysierflüssigkeit durch die Berechnung der Absorbanz bestimmen.

D.h. die jeweilige Fläche wird in die Absorbanz umgerechnet und ist somit proportional zur Konzentration. Ein punktweiser Vergleich ist ebenfalls möglich, weist aber eine geringere Messgenauigkeit auf. Die Absorbanz allgemein berechnet sich aus: (Detektor0 = 55000 wird beispielhaft als das Ausgangslevel mit reiner Dialysierflüssigkeit angenom-men)

$$\text{Absorbanz (A, B, C)} = \log10\ (\ \text{Detektor0}\ /\ \text{Detektorwert (effektiv)}\ )$$

Bolusmenge (arteriell) = 1 − (Absorbanz (C) / Absorbanz (A) ) * (Blutfluss * Messdauer)

Die gesamte Bestimmung der absoluten Rezirkulation erfolgt dann mit der folgenden Berechnungsformel:

$$Rezirkulation = \left( \frac{\left(1 - \left(\frac{\log_{10}\left(\frac{Detektor_0}{Detektorwert_B}\right)}{\log_{10}\left(\frac{Detektor_0}{Detektorwert_A}\right)}\right)\right) \cdot \left(Blutfluss \cdot (t_{2b} - t_{1b})\right)}{Bolusmenge\_ven\ddot{o}s} \right)$$

Zusammenfassend wird ein Verfahren und eine dieses implementierende Vorrichtung zur dialysierflüssigkeitsseitigen Erfassung einer Rezirkulation durch blutseitige Bolusgabe offenbart mit den folgenden Verfahrensschritten:

Spektralphotometrisches Messen eines ersten Licht - Absorbanzwerts (ad) einer von einem Dialysator abfließenden Dialysierflüssigkeit (d) vor Bolusgabe,
Venöse Bolusgabe mit vorbekanntem Volumen (QBven) am venösen Zugang, Spektralphotometrisches Messen eines zweiten Licht - Absorbanzwerts (bd) der abfließenden Dialysierflüssigkeit nach Bolusgabe und
Ermitteln einer Absorbanzwertänderung (Absorbanzdifferenz) zwischen dem ersten (ad) und zweiten gemessenen Absorbanzwerten (bd) infolge einer Bolus - Präsenz in der Dialysierflüssigkeit bei Rezirkulation als Grundlage für eine optionale Rezirkulationsquantifizierung.

**Patentansprüche**

1. Verfahren zur dialysierflüssigkeitsseitigen Erfassung einer Rezirkulation durch blutseitige Bolusgabe mit den folgenden Verfahrensschritten:

Spektralphotometrisches Messen eines ersten Licht - Absorbanzwerts (ad) einer von einem Dialysator (2) abfließenden Dialysierflüssigkeit (d) vor Bolusgabe,
Venöse Bolusgabe mit vorbekanntem Volumen (QBven) am venösen Zugang,
Spektralphotometrisches Messen eines zweiten Licht - Absorbanzwerts (bd) der abfließenden Dialysierflüssigkeit nach Bolusgabe und
Ermitteln einer Absorbanzswertänderung zwischen dem ersten (ad) und zweiten gemessenen Absorbanzwerten (bd) infolge einer Bolus - Präsenz in der Dialysierflüssigkeit bei Rezirkulation als Grundlage für die Rezirkulationsquantifizierung mit der Berechnungsformel:

$$\text{Rezirkulation} = \left( \frac{\left( 1 - \left( \dfrac{\log_{10}\left(\dfrac{Detektor_0}{Detektorwert_B}\right)}{\log_{10}\left(\dfrac{Detektor_0}{Detektorwert_A}\right)} \right) \right) \cdot \left( Blutfluss \cdot (t_{2b} - t_{1b}) \right)}{Bolusmenge\_ven\ddot{o}s} \right)$$

, wobei der Detektor 0 - Wert der Detektorwert der Kalibration ist, der Detektor B - Wert der Detektorwert mit Bolusgabe ist, der Detektor A - Wert der Detektorwert ohne Bolusgabe ist und (t2b - t1b) die Messdauer.

2. Verfahren nach Anspruch 1, **gekennzeichnet durch** die weiteren Verfahrensschritte:

   Integralbildung über den Absorbanz - Änderungsverlauf für die ersten und zweiten Absorbanzwerte (ad, bd) vor und nach dem Bolus,
   Differenzbildung zwischen den beiden Integralwerten (A, B) vor und nach dem Bolus und
   Identifizieren eines Rezirkulationszustands bei einer Differenz (C) ungleich null.

3. Verfahren nach Anspruch 2, **gekennzeichnet durch** die weiteren Verfahrensschritte:

   Umrechnen des Integralwerts (A) sowie der Integralwertdifferenz (C) in zugehörige Absorbanzwerte (AA, CA),
   Berechnen der arteriellen Bolusmenge (Qart) aus dem Verhältnis der Absorbanzwerte (AA, CA) und
   Berechnen des Rezirkulationsgrads aus dem Verhältnis der errechneten arteriellen Bolusmenge (QBart) zu der vorbekannten venösen Bolusmenge (QBven).

4. Verfahren nach einem der vorstehenden Ansprüche, **dadurch gekennzeichnet, dass** der Bolus keine Absorbanzeigenschaften bezüglich des in die Dialysierflüssigkeit emittierten Lichts hat.

5. Verfahren nach einem der Ansprüche 1 bis 3, **dadurch gekennzeichnet, dass** der Bolus vorbekannte Absorbanzeigenschaften bezüglich des in die Dialysierflüssigkeit emittierten Lichts vorzugsweise im Bereich der urämischen Toxine in der Dialysierflüssigkeit hat.

6. Dialysemaschine mit einer Vorrichtung zur dialysierflüssigkeitsseitigen Erfassung einer Rezirkulation durch blutseitige Bolusgabe
   **dadurch gekennzeichnet, dass**
   unter Implementierung des Verfahrens gemäß einem der Ansprüche 1 bis 5 mit einer Lichtquelle, die Licht mit einer Wellenlänge von 250 - 300nm und vorzugsweise 280nm emittiert und mindestens einem UV-Sensor (6), unmittelbar am Abfluss eines Dialysators (2), eine Recheneinheit, Sensorsignale bezüglich der UV - Absorbanz der Dialysierflüssigkeit vor und nach einer blutseitig venösen Bolusgabe erhält.

## Claims

1. A method of detecting a recirculation, at the side of a dialysis solution, by means of a blood-sided administration of a bolus, comprising the following method steps:

   measuring a first light absorbance value (ad) of a dialysis solution (d) draining off a dialyzer, by means of a spectrometer, prior to the administration of the bolus,
   venous administration of the bolus having a predefined volume (QBven) at the venous access,
   measuring a second light absorbance value (bd) of the draining dialysis solution, by means of a spectrometer, after the administration of the bolus and
   determining a change in the absorbance value between the first (ad) and second (bd) measured absorbance values due to the presence of a bolus in the dialysis solution during recirculation as a basis for the recirculation quantification, with the calculation formula

$$recirculation = \left( \frac{1 - \left( \frac{\log_{10}\left( \frac{detector_0}{detector\ value_B} \right)}{\log_{10}\left( \frac{detector_0}{detector\ value_A} \right)} \right) \cdot \left( blood\ flow \cdot (t_{2b} - t_{1b}) \right)}{bolus\ quantity\_venous} \right)$$

where the detector 0-value is the detector value of the calibration, the detector B-value is the detector value with administration of the bolus, the detector A-value is the detector value without administration of the bolus and (t2b-t1b) is the measurement duration.

2. The method according to claim 1, **characterized by** the further method steps:

determining the integral of the altered absorbance curve for the first and second absorbance values (ad, bd) prior to and after the bolus,
calculating the difference between the two integral values (A, B) prior to and after the bolus and
identifying a recirculation state if a difference (C) is unequal to zero.

3. The method according to claim 2, **characterized by** the further method steps:

converting the integral value (A) as well as the integral value difference (C) into associated absorbance values (AA, CA) by calculation,
calculating the arterial bolus quantity (Qart) from the ratio of the absorbance values (AA, CA) and
calculating the recirculation degree from the ratio between the calculated arterial bolus quantity (QBart) and the predefined venous bolus quantity (QBven).

4. The method according to any of the preceding claims, **characterized in that** the bolus does not have any absorbance characteristics with respect to the light emitted into the dialysis solution.

5. The method according to any of claims 1 to 3, **characterized in that** the bolus has predefined absorbance characteristics with respect to the light emitted into the dialysis solution, preferably in terms of the uremic toxins in the dialysis solution.

6. A dialysis apparatus comprising a device for detecting a recirculation, at the side of a dialysis solution, by means of a blood-sided administration of a bolus by implementation of the method according to any of the claims 1 to 5, comprising a light source which emits light with a wavelength from 250 to 300 nm and preferably of 280 nm, at least one UV sensor /6) directly at the drain of a dialyzer (2), and an arithmetic unit which receives sensor signal regarding the UV absorbance of the dialysis solution prior to and after a blood-sided venous administration of the bolus.

**Revendications**

1. Procédé de détection du dialysat d'une recirculation par l'administration de bolus sanguin comprenant les étapes de procédé suivantes :

mesure spectrophotométrique d'une première valeur d'absorbance lumineuse (ad) d'un dialysat (d) s'écoulant d'un dialyseur (2) avant l'administration de bolus,
administration de bolus par voie intraveineuse avec des volumes préalablement connus (QBven) lors de l'administration par voie intraveineuse,

mesure spectrophotométrique d'une seconde valeur d'absorbance lumineuse (bd) du dialysat s'écoulant après l'administration de bolus et

détermination d'une variation de la valeur d'absorbance entre la première (ad) et la seconde (bd) valeur d'absorbance mesurée en raison de la présence d'un bolus dans le dialysat lors de la recirculation comme base pour la quantification de la recirculation avec la formule de calcul :

$$\text{Recirculation} = \left( \frac{\left( 1 - \left( \frac{\log_{10}\left( \frac{\text{Détecteur}}{\text{Valeur de détecteur}_B} \right)}{\log_{10}\left( \frac{\text{Détecteur}_0}{\text{Valeur du détecteur}_A} \right)} \right) \right) \cdot (\text{débit sanguin} \cdot (t_{2b}-t_{1b}))}{\text{Quantité de bolus\_veineux}} \right)$$

, la valeur de détecteur 0 de la valeur de détecteur étant l'étalonnage, la valeur de détecteur B de la valeur de détecteur est avec administration de bolus, la valeur de détecteur A de la valeur de détecteur est sans administration de bolus et (t2b-t1b) est la durée de mesure.

2. Procédé selon la revendication 1, **caractérisé par** les étapes de procédé suivantes :

formation d'une intégrale via le gradient de variation de l'absorbance pour les première et seconde valeurs d'absorbance (ad, bd) avant et après le bolus,
formation d'une différentielle entre les deux valeurs d'intégrales (A, B) avant et après le bolus et
identification d'un état de recirculation pour une différentielle (C) non nulle.

3. Procédé selon la revendication 2, **caractérisé par** les étapes de procédé suivantes :

conversion de la valeur de l'intégrale (A), ainsi que la différentielle (C) de la valeur de l'intégrale en valeurs d'absorbance (AA, CA) correspondantes,
calcul de la quantité de bolus artériel (Qart) à partir du rapport des valeurs d'absorbance (AA, CA) et
calcul du degré de recirculation à partir du rapport de la quantité de bolus artériel calculée (QBart) sur la quantité de bolus veineux préalablement connue (QBven).

4. Procédé selon l'une quelconque des revendications précédentes, **caractérisé en ce que** le bolus ne présente pas de propriétés d'absorbance concernant la lumière émise dans le dialysat.

5. Procédé selon l'une quelconque des revendications 1 à 3, **caractérisé en ce que** le bolus présente des propriétés d'absorbance préalablement connues concernant la lumière émise dans le dialysat de préférence dans le domaine des toxines urémiques dans le dialysat.

6. Machine de dialyse comprenant un dispositif destiné à détecter un dialysat d'une recirculation par l'administration de bolus sanguin
**caractérisée en ce que**
lors de la mise en œuvre du procédé selon l'une quelconque des revendications 1 à 5 avec une source de lumière, la lumière émet avec une longueur d'onde de 250 à 300 nm et de préférence de 280 nm et au moins un capteur d'UV (6), directement à la sortie d'un dialyseur (2), une unité de calcul reçoit des signaux de capteur concernant l'absorbance des UV du dialysat avant et après une administration de bolus sanguin par voie intraveineuse.

EP 2 783 713 B1

**Fig. 1**

## Fig. 2

# Fig. 3

**Blutkreislauf**

Bolus

venöser Zugang

Patient

Rezirkulation 0%

arterieller Zugang

Blut-Pumpe 1

PBE

PV

Diaysator 2

**Dialysat**

DF-Pumpe 4

UV-Sensor 6

Abfluss

Fig. 4

**Dialysat**

**Blutkreislauf**

DF-Pumpe 4

Bolus

PV

venöser Zugang

Dialysator 2

Rezirkulation >0%

Patient

UV-Sensor 6

Abfluss

PBE

Blut-Pumpe 1

arterieller Zugang

EP 2 783 713 B1

Fig. 5

EP 2 783 713 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0900094 A
- US 7815852 B
- WO 9832477 A
- US 5685989 A
- DE 69916053 T2